# EUROPEAN PATENT APPLICATION

(11) **EP 3 388 052 A1**
(43) Date of publication of application: **17.10.2018**
(21) Application number: 16873301.2
(22) Date of filing: 05.12.2016
(51) Int. Cl.: A61K 8/98, A61K 8/46, A61K 8/97, A61K 8/64, A61K 8/41, A61Q 19/00

(54) **FUNCTIONAL COMPOSITION FOR SURFACE MODIFICATION**

(30) Priority: 09.12.2015 KR 20150175042
(71) Applicant: LG Household & Health Care Ltd., Seoul 03184 (KR)
(72) Inventor: SON, Seong Kil, Daejeon 34114 (KR); KIM, Dong Wan, Daejeon 34114 (KR); KIM, Kyung Hwan, Daejeon 34114 (KR); YOO, Ji Hee, Daejeon 34114 (KR); KIM, Jong Il, Daejeon 34114 (KR); LEE, Jeong Rae, Daejeon 34114 (KR); LEE, Sang Min, Daejeon 34114 (KR)
(74) Representative: Goddar, Heinz J.
(86) International application number: PCT/KR2016/014160
(87) International publication number: WO 2017/099436

(57) **Abstract**

The present invention relates to a functional composition for surface modification. The composition for surface modification, according to the present invention, comprises functional components having functional groups, which can be covalently bound to cysteine residues in proteins of hair, skin, fingernails or toenails, hide, or fiber, to form covalent bonds without damaging the hair, skin, fingernails or toenails, hide, or fiber, thereby remarkably improving the effects of the functional components, thus providing a desired surface modification effect semi-permanently.

## Description

### [Technical Field]

The present invention relates to a functional composition for surface modification of hair, skin, hide, or a fabric.

### [Background Art]

In general, hair, dead skin cells, fingernails, toenails, and wool fabrics mainly consist of keratin proteins, and anhydrous hair fibers are composed of about 90 to 97% proteins and about 2% lipids, and the remainder consists of nucleic acids, carbohydrates, and inorganic materials.

Hair is composed of about 50 wt% carbon, 7 wt% hydrogen, 22 wt% oxygen, 16 wt% nitrogen, and 5 wt% sulfur, as determined through an elemental analysis. As such, the major difference between the keratin proteins constituting hair and other common proteins is that a specifically large amount of the element sulfur is contained in amino acids constituting the protein. The specifically large amount of sulfur present in the keratin proteins of hair is known to play a very important role in maintaining the strength of hair protein by forming disulfide bonds (cysteine bonds) in hair.

The disulfide bonds present in hair are broken into cysteine through reduction accomplished by chemical treatment of hair, external environmental factors such as UV, or the like, and it is known that keratin of hair contains cysteine in an amount of about 16.6 to 18% of the total amino acids, whereas keratin of the epidermis contains cysteine only in an amount of about 2.3 to 3.8%, and collagen proteins of skin do not contain cysteine.

As such, the keratin proteins of hair have a significant difference from common proteins in terms of the content of cysteine. Wool fabrics made of wool are widely known to have a structure and composition of protein similar to those of human hair, and thus can be considered to contain a large amount of cysteine at a level similar to that in the proteins of hair.

In general, cosmetics, which are applied or spread on the human body, or used by other similar methods to cleanse and beautify the human body, promote attractiveness, change an appearance, or keep skin or hair healthy, mildly act on the human body.

Such cosmetics have various functions of washing, softening, imparting elasticity, volumizing, repairing damage, strengthening, dyeing, protecting, conditioning, bonding, blocking ultraviolet rays, suppressing and removing odors, antioxidizing, moisturizing, removing and alleviating blemishes, applying fragrances, removing and preventing dandruff and itching, perming, whitening, preventing wrinkles, stimulating and suppressing body hair growth, fingernail and toenail care, and oral cavity care, and also have functions of keeping various parts of the human body healthy and beautiful.

Examples of raw materials commonly used to constitute such cosmetics include oil-soluble raw materials such as oils and fats, waxes, hydrocarbons, higher fatty acids, higher alcohols, ester oils, silicone oils, and the like, polymer compounds used as anionic, cationic, amphoteric, or nonionic surfactants, whiteners, thickeners, or film formers, ultraviolet ray absorbents, ultraviolet ray blockers, antioxidants, chelating agents, coloring materials such as dyes or pigments, fragrances, preservatives, and the like.

In addition, examples of components for providing a specific functionality include oils and fats, natural or synthetic fatty acids, fatty alcohols, alcohols, alkyl glyceryl ethers, esters, hydrocarbons, silicones, fluorine compounds, polyhydric alcohols, sugars, natural or synthetic polymers, waxes, vitamins, hormones, amino acids, peptides, proteins, animal, plant, and mineral extracts, and derivatives thereof.

However, when hair, dead skin cells, fingernails, toenails, and wool fabrics are treated with such cosmetic components, it only provides a temporary and short-term effect until the next wash after the treatment, and the cosmetic component is completely washed away by the washing process so that the effect of the cosmetic component disappears. Therefore, there is always the inconvenience of re-treating hair, dead skin cells, fingernails, toenails, and wool fabrics with the cosmetic component.

In Korean Unexamined Patent Publication No. 2008-0064467, there is disclosed a non-aqueous personal care product for skin or hair. The personal care product for skin or hair includes a hair surface-modifying component and consists of a non-aqueous part and an aqueous part, wherein the non-aqueous part includes one or more selected from the group consisting of functional groups capable of covalently bonding with a protein residue in a hair or skin surface, such as carbonates, aldehydes, propionaldehyde, butylaldehyde, nitrophenyl carbonate, aziridines, isocyanate, thiocyanate, epoxides, tresylates, succinimide, hydroxysuccinimidyl esters, imidazole, oxycarbonylamidazole, imines, thiols, vinylsulfone, ethyleneimine, thioethers, acrylonitrile, acrylic or methacrylic acid ester, disulfides, ketones, and functional groups represented as RX (here, R is any one selected from the group consisting of alkyls, aryls, aralkyls, rings, and unsaturated rings, and X is I, Br, or Cl), and the non-aqueous part and the aqueous part are mixed immediately before using the product. However, various functional groups as described above are harmful to humans, or target, among amino acids of proteins constituting hair or skin, lysine contained in a small amount in hair and skin keratin (1.9 to 3.1% in hair and 3.1 to 6.9% in skin keratin) or cysteine contained in a small amount in hair and skin keratin (16.6 to 18% in hair and 2.3 to 3.8% in skin keratin) to participate in a reaction. Therefore, reaction efficiency is degraded, and each material needs to be synthesized separately.

### [Disclosure]

### [Technical Problem]

The present invention is directed to providing a functional composition for surface modification including a functional component having a functional group capable of covalently bonding with a thiol (-SH) group of cysteine in a molecule.

### [Technical Solution]

According to one embodiment of the present invention, there is provided a functional composition for surface modification including a functional component having a functional group capable of covalently bonding with a thiol (-SH) group of cysteine in a molecule.

According to another embodiment of the present invention, there is provided a functional composition for surface modification including a functional component having a functional group capable of covalently bonding with a thiol (-SH) group of cysteine in a molecule; and a cystine reducing agent.

According to still another embodiment of the present invention, there is provided a care product including the functional composition for surface modification.

### [Advantageous Effects]

A composition for surface modification according to the present invention includes a functional component having a functional group capable of covalently bonding with a cysteine residue in the protein of hair, skin, fingernails, toenails, a hide, or a fabric to form covalent bonds in hair, skin, fingernails, toenails, a hide, or a fabric so that the effect and persistence of the functional component are remarkably improved, and thereby a desired functional effect can be maximized semi-permanently.

### [Best Mode]

The present invention relates to a functional composition for surface modification including a functional component having a functional group capable of covalently bonding with a thiol (-SH) group of cysteine in a molecule.

The term "functionality" used herein refers to an effect of washing, softening, imparting elasticity, volumizing, enhancing thickness, preventing and repairing damage, strengthening, dyeing, protecting, imparting gloss, coating, conditioning, bonding, blocking ultraviolet rays, suppressing and removing odors, antioxidizing, moisturizing, removing and alleviating scalp blemishes, applying fragrances, removing and preventing dandruff and itching in the scalp, perming, whitening, preventing wrinkles, stimulating and suppressing body hair growth, and resisting bacteria and injurious insects as well as a care effect on a protein material containing a specifically large amount of sulfur, such as a hide, fingernails, and toenails.

The term "functional composition for surface modification" may encompass a functional composition for hair surface modification, a functional composition for skin, fingernail or toenail surface modification, a functional composition for hide surface modification, and/or a functional composition for fabric surface modification.

The term "hair surface modification" used herein refers to changing the properties of a hair surface by coating hair or bonding an active ingredient with hair. The functional composition for hair surface modification may be applied to the hair, spread on the hair, or used by other similar methods, so that it serves to impart beneficial properties to the hair through conditioning effects such as making hair appear thick, enhancing gloss, softening, eliminating frizz, enhancing smoothness, and the like so as to beautify the hair, promote attractiveness, and change an appearance; moisturize the hair; or block ultraviolet rays to keep the hair healthy. For example, hair surface modification effects of reinforcing hair tensile strength, enhancing hair thickness, and the like may be exhibited.

The term "skin, fingernail, toenail, or hide surface modification" used herein refers to changing the properties of a skin, fingernail, toenail, or hide surface by coating the skin, fingernails, toenails, or a hide or bonding an active ingredient with the skin, fingernails, toenails, or hide. The functional composition for the skin, fingernail, toenail, or hide surface modification may be applied to the skin, fingernails, toenails, or hide, spread on the skin, fingernails, toenails, or hide, or used by other similar methods, so that it serves to impart beneficial properties to the skin, fingernails, toenails, or hide through conditioning effects such as making skin, fingernails, toenails, or hide look bright, enhancing gloss, softening, enhancing smoothness, and the like so as to beautify the skin, fingernails, toenails, or hide, promote attractiveness, and change an appearance; moisturize the skin, fingernails, toenails, or hide; or block ultraviolet rays to keep the skin, fingernails, toenails, or hide healthy and strong. For example, skin surface modification effects of improving skin wrinkles, enhancing moisture in skin, and the like may be exhibited, fingernail and toenail surface modification effects of supplying nutrition to fingernails and toenails, preventing fingernails and toenails from being colored and split, and the like may be exhibited, and hide surface modification effects of enhancing gloss, softening hide, and the like may be exhibited.

The term "fabric surface modification" used herein refers to changing the properties of a fabric surface by coating a fabric such as wool, silk, or the like or bonding an active ingredient with the fabric. The functional composition for fabric surface modification may be applied to a fabric, spread on a fabric, or used by other similar methods, so that it serves to impart beneficial properties to the fabric through effects such as preventing damage to the fabric, maintaining a fragrance for a long time, bleaching and sterilizing the fabric, blocking ultraviolet rays, and softening the fabric so as to retain the fabric as new. For example, fabric surface modification effects of enhancing softness of a fabric, maintaining the original feeling of color of a fabric for a long time, and the like may be exhibited.

The functional component is a material having the functional effects described above, and may be, specifically, one or more selected from the group consisting of natural extracts, amino acids, peptides, proteins, polymers, silicones, fatty alcohols, fatty acids, waxes, esters, ethers, amines, quaternary ammonium, hydrocarbons, alky glyceryl esters, alcohols, polyhydric alcohols, sugars, surfactants, derivatives of ethylene or polyethylene, polypropylene, powders, dyes, antimicrobial agents, insect repellents, fragrances, vitamins, and derivatives thereof. Also, any component capable of modifying a hair, skin, fingernail, toenail, hide, or fabric surface may be used as the functional component.

The natural extract may be a *Rhodiola rosea* extract, a *Camellia japonica* leaf extract, ursolic acid, a *Rhus javanica* extract, an algae extract, a *Helianthus annuus* seed extract, a *Sophora flavescens* extract, a *Panax ginseng* root extract, a *Coptis chinensis* root extract, a *Calendula officinalis* extract, *Betula platyphylla* sap, a *Betula alba* extract, a *Zanthoxylum bungeanum maxim* extract, a *Luffa cylindrical* extract, a *Monarda didyma* extract, a *Chamaecyparis obtusa* extract, a *Rhodiola rosea* extract, a *Sophora flavescens* extract, an *Atractylodes* rhizome extract, a *Centella asiatica* extract, a *Coptis chinensis* extract, red ginseng root water, a *Fritillaria ussuriensis* extract, a *Convallaria keiskei* extract, a honeycomb extract, a cassis extract, a pomegranate extract, a lemon extract, a pine bud extract, a green tea extract, a broccoli extract, a honey extract, a cranberry extract, a berry extract, a lavender extract, a lentil extract, a ginger extract, or the like. The protein and the peptide may be a protein and a peptide which are obtained from Chun-zam silk, silk, polylysine, algae, wool, hair, or wheat, or a derivative thereof.

The amino acid may be glycine, alanine, valine, leucine, isoleucine, threonine, serine, cysteine, cystine, methionine, aspartic acid, asparagine, glutamic acid, diiodotyrosine, lysine, arginine, histidine, phenylalanine, tyrosine, tryptophan, proline, oxyproline, or a derivative thereof.

The peptide may be a peptide consisting of 2 to 200 amino acids, and the protein may be keratin, collagen, gelatin, a vegetable protein, a hydrolyzed vegetable protein, or a derivative thereof. The keratin is preferably hydrolyzed keratin, and more preferably has a molecular weight of 200 to 150,000 Da.

The protein may include active enzymes and all inactive proteins, which are obtained from natural microorganisms, animals, or plants or obtained through genetic modification and manipulation of microorganisms, a derivative thereof, a hydrolysate thereof, and a protein modified therefrom. For example, the protein may be a transglutaminase, a protease, a lipase, a hydrolyzed keratin protein, a hydrolyzed collagen protein, a hydrolyzed silk protein, a hydrolyzed milk protein, a hydrolyzed pearl protein, a hydrolyzed plant (wheat, soybean, sesame, etc.) protein, or a derivative thereof, but the present invention is not limited thereto.

The polymer may be a linear/branched chain-type or network-type polymer compound having a molecular weight of about 1,000 to 1,000,000, may include a double bond or various ring-structural substituents between carbon atoms as necessary, and preferably includes in the molecule at least one residue with reactivity with a bioreactive functional group such as -COONa, -COOK, -COOH, -NH₂, -NHR, -NR₂, -Cl, -Br, -I, or -F for easily attaching the bioreactive functional group to any one terminus of the molecule. More preferably, the polymer is a compound which is a linear/branched chain-type polymer having about 10,000 to 500,000 carbon atoms and includes in the molecule at least one residue with reactivity with a bioreactive functional group such as -COONa, -COOK, -COOH, -NH₂, -NHR, -NR₂, -Cl, -Br, -I, or -F for easily attaching the bioreactive functional group to any one terminus of the molecule. For example, the polymer may be an amphoteric polymer such as an amino acid polymer (e.g., polylysine), a polyamine polymer, a polycarboxylic acid polymer, a methacryloyl ethyl betaine/methacrylate copolymer, or an octylacrylamide/acrylate/butylaminoethyl methacrylate copolymer; a nonionic polymer such as polyvinylpyrrolidone (PVP), a PVP/vinyl acetate (VA) copolymer, a PVP/dimethylaminoethyl methacrylate copolymer, or polyurethane; or an anionic polymer such as an acrylate/methacrylate copolymer or a VA/crotonate/vinyl neodecanoate copolymer, but the present invention is not limited thereto.

The silicone may be, for example, a compound such as dimethicone, trimethicone, phenyl trimethicone, amodimethicone, amodi phenyl trimethicone, amodi-penta phenyl trimethicone, dimethylpolysiloxane, methylphenylpolysiloxane, decamethylcyclopentasiloxane, methyl trimethicone, phenyl trimethicone, methicone, cyclomethicone, an alkyl methyl siloxane, dimethicone copolyol, or trimethylsilylamodimethicone, but the present invention is not limited thereto.

The fatty alcohol is a C10 to C50 linear/branched fatty alcohol compound, and preferable examples thereof include lauryl alcohol, cetyl alcohol, stearyl alcohol, isostearyl alcohol, behenyl alcohol, and the like, but the present invention is not limited thereto.

The fatty acid is a C10 to C50 linear/branched fatty acid compound, and preferable examples thereof include 18-methyl eicosanoic acid, lauric acid (dodecanoic acid), stearic acid, isostearic acid, and the like, but the present invention is not limited thereto.

The wax may be, for example, candelilla wax, carnauba wax, ricebran wax, beeswax, lanoline, ozokerite, ceresin wax, paraffin wax, microcrystalline wax, polyethylene wax, or the like, but the present invention is not limited thereto.

The ester may be, for example, isopropyl myristate, butyl myristate, isopropyl palmitate, ethyl stearate isopropyl linoleate, decyl myristate, cetyl myristate, cetyl palmitate, hydrogenated polyisobutene, or the like, but the present invention is not limited thereto.

In the present invention, a material having a functional group capable of covalently bonding with one or more proteins and selected from the group consisting of amines, imidoesters, aryl azides, diazirines, hydroxymethyl phosphine, pentafluorophenyl esters, pyridyl disulfide, sulfo-hydroxysuccinimide esters, alkoxyamines, hydrazides, haloacetyls, azide, carbonates, aldehydes, propionaldehyde, butylaldehyde, nitrophenyl carbonate, aziridines, isocyanate, thiocyanate, epoxides, tresylates, succinimide, hydroxysuccinimidyl esters, imidazole, oxycarbonylamidazole, imines, thiols, vinylsulfone, ethyleneimine, thioethers, acrylonitrile, acrylic/carboxylic/methacrylic acid esters, disulfides, and ketones may be additionally used.

In the present invention, the functional group capable of covalently bonding with a thiol (-SH) group of cysteine may be a compound including at least one residue of an alkene, a vinyl group, or an alkyne, which is represented by the following Chemical Formulas 1 to 7, in a molecule.

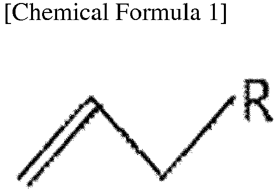

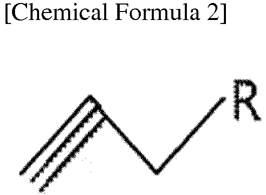

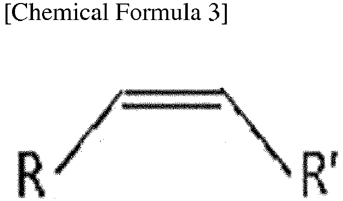

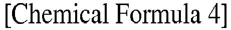

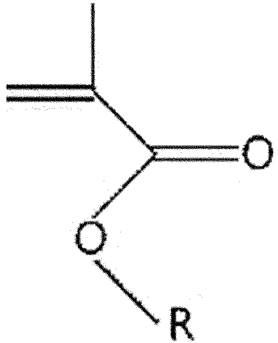

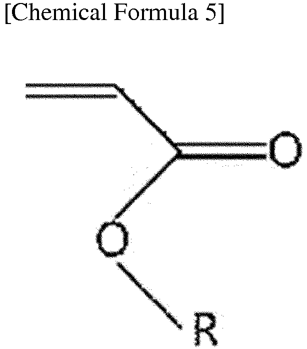

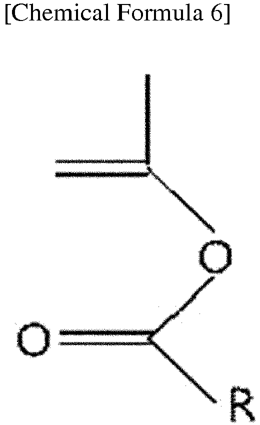

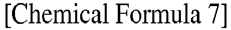

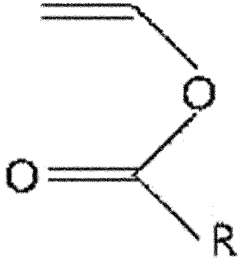

In Chemical Formulas 1 to 7, R or R' represents a C1 to C60 linear, branched, or cyclic (including elements such as O, N, Si, and the like and a double bond in a ring) hydrocarbon or aromatic hydrocarbon, wherein a portion of the hydrocarbon molecule includes a double bond, is substituted with one or more elements selected from the group consisting of O, N, S, P, and Si, is substituted in an anionic, cationic, or amphoteric form, or is bonded with a metal ion in a salt form, but the present invention is not limited thereto.

The functional component having a functional group capable of covalently bonding with a thiol (-SH) group of cysteine in a molecule may be, for example, one or more selected from the group consisting of [2-(acryloyloxy)ethyl] trimethyl ammonium chloride, which is a quaternary ammonium salt; pentaerythritol tri(allyl ether) and trimethylolpropane di(allyl ether), which are ethers; phosphoric acid 2-hydroxyethyl methacrylate ester, hexanedioic acid divinyl ester, and 3,6,9-trioxaundecanedioic acid divinyl ester, which are esters; butanediol diacrylate and butanediol dimethacrylate, which are alcohols; ethylene glycol methyl ether acrylate, ethylene glycol methyl ether methacrylate, poly(ethylene glycol) methyl ether acrylate, poly(ethylene glycol) methyl ether methacrylate, trimethylolpropane triacrylate, pentaerythritol tetraacrylate, dipentaerythritol pentaacrylate, tris(2-hydroxy ethyl) isocyanurate triacrylate, tetra(ethylene glycol) diacrylate, tetra(ethylene glycol) dimethacrylate, polyethylene glycol acrylate, polyethylene glycol diacrylate, polyethylene glycol triacrylate, polyethylene glycol tetraacrylate, polyethylene glycol pentaacrylate, polyethylene glycol hexaacrylate, polyethylene glycol heptaacrylate, polyethylene glycol octaacrylate, polyethylene glycol methacrylate, polyethylene glycol dimethacrylate, polyethylene glycol trimethacrylate, polyethylene glycol tetramethacrylate, polyethylene glycol pentamethacrylate, polyethylene glycol hexamethacrylate, polyethylene glycol heptamethacrylate, and polyethylene glycol octamethacrylate, which are derivatives of ethylenes or polyethylenes; vinyl silicones and alkyne silicones, which are silicones; and derivatives thereof, but the present invention is not limited thereto.

The functional component having a functional group capable of covalently bonding with a thiol (-SH) group of cysteine in a molecule may be used in an amount of 0.0001 to 50 parts by weight, 0.001 to 30 parts by weight, 0.01 to 10 parts by weight, or 0.1 to 5 parts by weight with respect to 100 parts by weight of the entire composition. When the content of the functional component is less than 0.0001 part by weight, there is a limitation in providing the effect of an active component, and when the content of the functional component is greater than 50 parts by weight, there is a problem in formulation and the stability of formulation over time, and the component may be lost without reacting.

In order to increase the effect of the functional component, the functional composition according to the present invention may also include a cystine reducing agent in addition to the functional component having a functional group capable of covalently bonding with a thiol (-SH) group of cysteine in a molecule, so that the content of cysteine in hair, skin, fingernails, toenails, a hide, and/or a fabric, which is capable of reacting with an alkene, a vinyl group, or an alkyne, may increase.

Therefore, the present invention provides the functional composition for surface modification including the functional component having a functional group capable of covalently bonding with a thiol (-SH) group of cysteine in a molecule and the cystine reducing agent.

The cystine reducing agent serves to break a disulfide bond (-S-S-) into cysteine (-SH) through reduction, and may be, specifically, one or more selected from the group consisting of ascorbic acid, erythorbic acid, sodium ascorbate, potassium ascorbate, calcium ascorbate, ammonium ascorbate, monoethanolamine ascorbate, diethanolamine ascorbate, sodium erythorbate, disodium ascorbyl sulfate, disodium erythorbyl sulfate, magnesium ascorbyl phosphate, ascorbyl palmitate, ascorbyl stearate, ascorbyl dipalmitate, ascorbyl tetra 2-hexyl decanoate, ascorbyl myristate, ascorbyl laurate, ascorbyl acetate, ascorbyl propionate, ascorbyl tartrate, ascorbyl citrate, ascorbyl succinate, ascorbyl benzoate, potassium (ascorbyl/tocopheryl) phosphate, ethyl ascorbic acid, allantoin ascorbate, chitosan ascorbate, methylsilanol ascorbate, ascorbyl tetradecyl hexyl, aminopropyl ascorbyl phosphate, ascorbic acid polypeptide, ascorbyl glucoside, ascorbyl methylsilanol pectinate, thioglycolic acid, thioglycolate, thioglycolic acid ester, ammonium thioglycolate, sodium thioglycolate, glyceryl thioglycolate, cysteine, cysteine hydrochloride, N-acetyl-L-cysteine, thioglycerol, thiolacetic acid, thiomalic acid, cysteamine, sulfurous acid, ammonium sulfite, sodium sulfite, ammonium hydrogensulfite, sodium hydrogensulfite, thiosulfuric acid, sodium thiosulfate, cystamine, N,N'-bisacryloyl-cystamine, 3,3'-dithiopropionic acid, glutathione disulfide, cysteine hydrochloride, N-acryloyl-cysteamine, 2-iminothiolane, N-acetylhomocysteine thiolactone, 1,4-dithiothreitol, glutathione, and salts and derivatives thereof, but the present invention is not limited thereto. In particular, it is most preferable that cysteine hydrochloride be used as the cystine reducing agent to prevent damage caused by excessive reduction, but a type of the reducing agent is not limited thereto.

The cystine reducing agent may be used in an amount of 0.0001 to 20 parts by weight, 0.001 to 10 parts by weight, 0.01 to 5 parts by weight, or 0.1 to 3 parts by weight with respect to 100 parts by weight of the entire composition. When the content of the cystine reducing agent is less than 0.0001 part by weight, there may be a problem in actual reduction of cystine due to an excessively low content, and when the content of the cystine reducing agent is greater than 20 parts by weight, a substrate to be treated may be damaged, the stability of a formulation may be degraded, and the contents may have bad odor due to excessive reduction.

The following Reaction Scheme 1 shows a schematic diagram of a reaction according to one embodiment of the present invention, in which [2-(acryloyloxy)ethyl] trimethyl ammonium chloride, which is an antimicrobial functional component having a vinyl group as a functional group reactive with cysteine in a molecule, and cysteine hydrochloride, which is a reducing agent, react with hair to reduce cystine, and thus covalent bonds between cysteine in hair and the cosmetic component are formed.

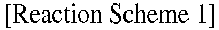

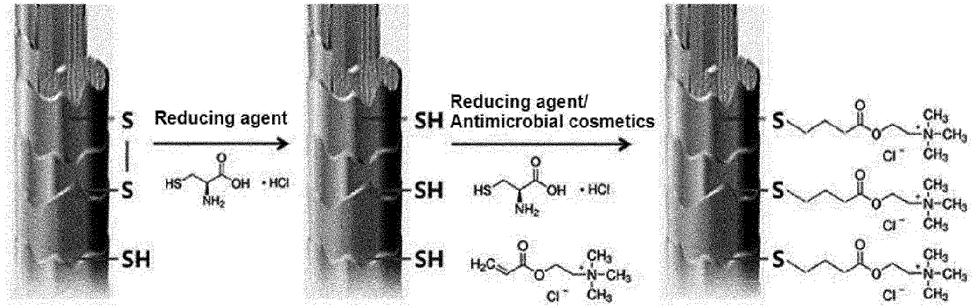

Reaction Scheme 1 illustrates a method in which a reducing agent is applied first to reduce a disulfide bond, and a reducing agent and a functional component are applied together at the same time, which is used to form covalent bonds.

The cysteine-reactive functional component bonded to hair, skin, fingernails, toenails, a hide, and/or a fabric may not be easily detached during general washing with a shampoo, a detergent, a soap, or the like, but may be almost permanently attached to the hair, skin, fingernails, toenails, a hide, and/or fabric.

In order to additionally increase an effect of the composition for surface modification according to the present invention, the composition may be used in combination with a fatty acid (palmitic acid, stearic acid, and the like), a fatty alcohol, a cationized polymer such as a cationic surfactant (linear/branched long-chain alkyl quaternary ammonium salts or the like), cationic cellulose, cationic guar, cationic polyvinylpyrrolidone, and the like, a silicone, or the like for ease of formulation. Also, in order to formulate the composition as a cosmetic, components such as a solvent, a surfactant, a thickening agent, a stabilizer, a preservative, a coloring agent, a pH controlling agent, a chelating agent, a coloring agent, a pearlescent agent, an appearance improving agent, a pigment, a particulate material, and the like may be additionally included. The components for formulation may be used in an amount of 40 to 99 parts by weight with respect to 100 parts by weight of the entire composition.

The composition for surface modification according to the present invention may be used in the treatment with a care product for hair, skin, fingernail, toenail, hide, or fabric surface modification.

For hair, any one of cosmetic formulations that can be used for hair, such as pre-shampoo products, shampoos, conditioners, hair treatments, waxes, sprays, mousses, hair lotions, essences, hair creams, packs, masks, sheets, tablets, patches, strips, ointment-type products, permanent dyes, temporary dyes, hair perm products, and the like, may be used. For skin, any one of cosmetic formulations that can be used for skin, such as body and facial cleansers, softeners, toners, lotions, essences, serums, creams, gels, foundations, powders, makeup bases, point makeup products, masks, patches, and the like, may be used. For fingernails and toenails, any one of fingernail and toenail care formulations that can be used for fingernails and toenails, such as nail varnishes, coats, nail polishes, coloring agents, nail hardeners, therapeutic agents, nail softeners, and the like, may be used. For a hide, any one of hide care formulations that can be used for a hide, such as coats, cleaners, softeners, polishes, and the like, may be used. For a fabric, any one of fabric care formulations that can be used for a fabric, such as fabric softeners, fabric dyes, laundry detergents, fabric treatments, pre- and post-treating agents, laundry aids, and the like, may be used.

More preferably, the composition for surface modification according to the present invention includes a functional component. If the activity of the formulation is degraded when the formulation is in an aqueous environment, it may be beneficial if the formulation takes a non-aqueous form to maintain its activity, which may be achieved by inducing a reaction of the formulation by mixing it with a buffer solution immediately before use to adjust a pH or bringing it in contact with water in a washing process. Examples of a non-aqueous form include liquid-phase solubilized products, liquid-phase emulsions, liquid-phase dispersed products, packs, masks, sheets, tablets, patches, strips, ointment-type products, formulations containing capsules, powder-type products, tablet-type products, oils, waxes, ampoules, gels, and the like, which are commonly used as non-aqueous cosmetic formulations. In order to increase the effect of the functional component in the composition for surface modification according to the present invention, a dibasic acid ester such as dioctyl succinate, dioctyl adipate, diethyl sebacate, or the like, a polyol, polyethylene glycol, propylene glycol, hexylene glycol, butanediol and an isomer thereof, glycerol, benzyl alcohol, or ethoxydiglycol and a derivative thereof may be used. The solvent mentioned above serves to increase permeability into the hair and skin and is used as a solvent for a poorly soluble material.

As described above, a component for surface modification having two or more reactive functional groups reacts with a hair, skin, fingernail, toenail, hide, or fabric surface to form a surface-modifying layer with a multi-layer structure, which may result in increasing reaction efficiency. Also, a functional component having a functional group capable of covalently bonding with the protein in a hair, skin, fingernail, toenail, hide, or fabric surface may be additionally used and reacted with the outermost surface of the surface-modifying layer to provide an additional effect besides a surface modification effect.

### [Modes of the Invention]

Hereinafter, the present invention will be described in detail with reference to the following embodiments. However, the following embodiments are merely presented to exemplify the present invention, and the content of the present invention is not limited to the following embodiments.

### Comparative Examples 1 and 2 and Examples 1 to 9: Hair shampoo composition

Compositions for a hair shampoo were prepared in compositions and quantities listed in the following Table 1.

First, polyquaternium-10, which is a cationic polymer, was dispersed in purified water being stirred, EDTA 4Na, which is a chelating agent, was added thereto, and then the mixture was stirred until it became transparent. Afterward, sodium lauryl ether (2 moles) sulfate, which is an anionic surfactant, and cocamidopropyl betaine, which is an amphoteric surfactant, were added to the mixture, followed by stirring for 20 minutes or more. Then, a fragrance and sodium ascorbate, cysteine hydrochloride, [2-(acryloyloxy)ethyl] trimethyl ammonium chloride, and polyethylene glycol acrylate, which are components according to the present invention, were added to prepare a shampoo.

Compositions containing zinc pyrithione commonly used as a bactericide against dandruff bacteria in anti-dandruff shampoos (Comparative Example 1); propylene glycol commonly used as a moisturizing component in shampoos (Comparative Example 2); either [2-(acryloyloxy)ethyl] trimethyl ammonium chloride having an antimicrobial effect or polyethylene glycol acrylate having a moisturizing effect, which are components capable of surface modification according to the present invention, but no cystine reducing agent (respectively, Examples 1 and 2); sodium ascorbate as a cystine reducing agent, and either [2-(acryloyloxy)ethyl] trimethyl ammonium chloride or polyethylene glycol acrylate (respectively, Examples 3 and 4); cysteine hydrochloride as a cystine reducing agent, and either [2-(acryloyloxy)ethyl] trimethyl ammonium chloride or polyethylene glycol acrylate (respectively, Examples 5 and 6); both sodium ascorbate and cysteine hydrochloride as cystine reducing agents, and either [2-(acryloyloxy)ethyl] trimethyl ammonium chloride or polyethylene glycol acrylate (respectively, Examples 7 and 8); and both sodium ascorbate and cysteine hydrochloride as cystine reducing agents, and both [2-(acryloyloxy)ethyl] trimethyl ammonium chloride and polyethylene glycol acrylate (Example 9) were prepared.

**[Table 1]**

| Composit ion (wt%) | Comparati ve Example 1 | Compa rative Examp le 2 | Examp le 1 | Examp le 2 | Examp le 3 | Examp le 4 | Examp le 5 | Examp le 6 | Examp le 7 | Examp le 8 | Examp le 9 |
|---|---|---|---|---|---|---|---|---|---|---|---|
| Water | 43.55 | 43.55 | 43.55 | 43.55 | 42.55 | 42.55 | 42.55 | 42.55 | 42.55 | 42.55 | 37.55 |
| Polyquate mium-10 | 0.5 | 0.5 | 0.5 | 0.5 | 0.5 | 0.5 | 0.5 | 0.5 | 0.5 | 0.5 | 0.5 |
| EDTA 4Na | 0.05 | 0.05 | 0.05 | 0.05 | 0.05 | 0.05 | 0.05 | 0.05 | 0.05 | 0.05 | 0.05 |
| Sodium lauryl ether (2 moles) sulfate (30%) | 35 | 35 | 35 | 35 | 35 | 35 | 35 | 35 | 35 | 35 | 35 |
| Cocamido propyl betaine (30%) | 15 | 15 | 15 | 15 | 15 | 15 | 15 | 15 | 15 | 15 | 15 |
| Fragrance | 0.9 | 0.9 | 0.9 | 0.9 | 0.9 | 0.9 | 0.9 | 0.9 | 0.9 | 0.9 | 0.9 |
| Zinc pyrithione (50%) | 5 | - | - | - | - | - | - | - | - | | |
| Propylene glycol | - | 5 | - | - | - | - | - | - | - | - | - |
| Sodium ascorbate | - | - | - | - | 1 | 1 | - | - | 0.5 | 0.5 | 0.5 |
| Cysteine hydrochlo ride | - | - | - | - | - | - | 1 | 1 | 0.5 | 0.5 | 0.5 |
| [2-(Acryloyl oxy)ethyl ] trimethyl ammoniu m chloride (antimicr obial effect) | - | - | 5 | - | 5 | - | 5 | - | 5 | - | 5 |
| Polyethyl ene glycol acrylate (moisturiz ing effect) | - | - | - | 5 | - | 5 | - | 5 | - | 5 | 5 |
| Total | 100.00 | 100.00 | 100.00 | 100.00 | 100.00 | 100.00 | 100.00 | 100.00 | 100.00 | 100.00 | 100.00 |

Each of the shampoo compositions according to Comparative Examples 1 and 2 and Examples 1 to 9 was used to wash a hair tress having a length of 12 cm and a weight of 3 g ten times to induce surface modification, and then each tress was washed three times with a 15% sodium lauryl ethyl sulfate solution (cleansing surfactant) to remove antimicrobial and moisturizing components which were not covalently bonded but merely adsorbed onto the hair. Afterward, the hair tresses thus treated were compared in terms of bactericidal and moisturizing effects, the results of which are shown in the following Table 2 (bactericidal ability against dandruff bacteria) and Table 3 (hair moisturizing effect).

**[Table 2]**

| Classification | Comparative Example 1 | Example 1 | Example 3 | Example 5 | Example 7 | Example 9 |
|---|---|---|---|---|---|---|
| Bactericidal ability against dandruff bacteria (24 hours, %) | 10.5 | 95.0 | 99.5 | 99.0 | 97.5 | 98.5 |

**[Table 3]**

| Classification | Comparative Example 2 | Example 2 | Example 4 | Example 6 | Example 8 | Example 9 |
|---|---|---|---|---|---|---|
| Water contained in hair (%) | 10.3 | 19.2 | 25.7 | 24.3 | 20.6 | 22.3 |

As shown in Tables 2 and 3, it was confirmed that Examples 1 to 9 containing the components according to the present invention exhibited excellent bactericidal and moisturizing effects even after the hair was washed three times with a cleansing surfactant because the hair surface was modified by bactericidal and moisturizing components covalently bonded to the hair surface.

### Comparative Examples 3 and 4 and Examples 10 to 18: Moisturizing and bactericidal skin lotion composition

Compositions for moisturizing and bactericidal skin lotions were prepared through a common method of preparing a lotion in compositions and quantities as listed in the following Table 4.

Compositions containing phenoxyethanol commonly used as a bactericide in bactericidal and moisturizing agents for skin (Comparative Example 3); propylene glycol commonly used as a skin-moisturizing component in such agents (Comparative Example 4); either [2-(acryloyloxy)ethyl] trimethyl ammonium chloride having an antimicrobial effect or polyethylene glycol acrylate having a moisturizing effect, which are components capable of surface modification according to the present invention, but no cystine reducing agent (respectively, Examples 10 and 11); sodium ascorbate as a cystine reducing agent, and either [2-(acryloyloxy)ethyl] trimethyl ammonium chloride or polyethylene glycol acrylate (respectively, Examples 12 and 13); cysteine hydrochloride as a cystine reducing agent, and either [2-(acryloyloxy)ethyl] trimethyl ammonium chloride or polyethylene glycol acrylate (respectively, Examples 14 and 15); both sodium ascorbate and cysteine hydrochloride as cystine reducing agents, and either [2-(acryloyloxy)ethyl] trimethyl ammonium chloride or polyethylene glycol acrylate (respectively, Examples 16 and 17); and both sodium ascorbate and cysteine hydrochloride as cystine reducing agents, and both [2-(acryloyloxy)ethyl] trimethyl ammonium chloride and polyethylene glycol acrylate (Example 18) were prepared.

**[Table 4]**

| Composition (wt%) | Comparative Example 3 | Comparative Example 4 | Example 10 | Example 11 | Example 12 | Example 13 | Example 14 | Example 15 | Example 16 | Example 17 | Example 18 |
|---|---|---|---|---|---|---|---|---|---|---|---|
| Water | 78.3 | 74.3 | 78.3 | 74.3 | 78.1 | 74.1 | 78.1 | 74.1 | 78.1 | 74.1 | 73.1 |
| Glycerine | 3 | 3 | 3 | 3 | 3 | 3 | 3 | 3 | 3 | 3 | 3 |
| Butylene glycol | 3 | 3 | 3 | 3 | 3 | 3 | 3 | 3 | 3 | 3 | 3 |
| Carboxyviny 1 polymer | 0.1 | 0.1 | 0.1 | 0.1 | 0.1 | 0.1 | 0.1 | 0.1 | 0.1 | 0.1 | 0.1 |
| Polysorbate 60 | 1.5 | 1.5 | 1.5 | 1.5 | 1.5 | 1.5 | 1.5 | 1.5 | 1.5 | 1.5 | 1.5 |
| Sorbitan sesquioleate | 1.5 | 1.5 | 1.5 | 1.5 | 1.5 | 1.5 | 1.5 | 1.5 | 1.5 | 1.5 | 1.5 |
| Liquid paraffin | 0.5 | 0.5 | 0.5 | 0.5 | 0.5 | 0.5 | 0.5 | 0.5 | 0.5 | 0.5 | 0.5 |
| Carprylic/ca pric triglyceride | 5 | 5 | 5 | 5 | 5 | 5 | 5 | 5 | 5 | 5 | 5 |
| Triethanolam ine | 0.1 | 0.1 | 0.1 | 0.1 | 0.1 | 0.1 | 0.1 | 0.1 | 0.1 | 0.1 | 0.1 |
| Stearyl alcohol | 5 | 5 | 5 | 5 | 5 | 5 | 5 | 5 | 5 | 5 | 5 |
| Fragrance | 1 | 1 | 1 | 1 | 1 | 1 | 1 | 1 | 1 | 1 | 1 |
| Phenoxyetha nol | 1 | - | - | - | - | - | - | - | - | - | - |
| Propylene glycol | - | 5 | - | - | - | - | - | - | - | - | - |
| Sodium ascorbate | - | - | - | - | 0.2 | 0.2 | - | - | 0.1 | 0.1 | 0.1 |
| Cysteine hydrochlorid e | - | - | - | - | - | - | 0.2 | 0.2 | 0.1 | 0.1 | 0.1 |
| [2-(Acryloyloxy )ethyl] trimethyl ammonium chloride | - | - | 1 | - | 1 | - | 1 | - | 1 | - | 1 |
| Polyethylene glycol acrylate | - | - | - | 5 | - | 5 | - | 5 | - | 5 | 5 |
| Total | 100.0 | 100.0 | 100.0 | 100.0 | 100.0 | 100.0 | 100.0 | 100.0 | 100.0 | 100.0 | 100.0 |

Each of the bactericidal and moisturizing compositions according to Comparative Examples 3 and 4 and Examples 10 to 18 was used to treat a pig skin sample with a size of 1 cm x 1 cm once, followed by washing of the sample with water, for total of ten times to induce skin surface modification. Afterward, each skin sample was washed three times with a 15% sodium lauryl ethyl sulfate solution (cleansing surfactant) to remove antimicrobial and moisturizing components which were not covalently bonded but merely adsorbed onto the skin, and the pig skin samples thus treated were compared in terms of bactericidal and moisturizing effects, the results of which are shown in the following Table 5 (bactericidal ability against E. coli) and Table 6 (skin moisturizing effect).

**[Table 5]**

| Classification | Comparative Example 3 | Example 10 | Example 12 | Example 14 | Example 16 | Example 18 |
|---|---|---|---|---|---|---|
| Bactericidal ability against E. coli (24 hours, %) | 12.5 | 96.0 | 99.0 | 99.0 | 98.0 | 98.0 |

**[Table 6]**

| Classification | Comparative Example 4 | Example 11 | Example 13 | Example 15 | Example 17 | Example 18 |
|---|---|---|---|---|---|---|
| Water contained in skin (%) | 9.3 | 20.6 | 27.2 | 28.6 | 25.3 | 26.0 |

As shown in Tables 5 and 6, it was confirmed that Examples 10 to 18 containing the components according to the present invention exhibited excellent bactericidal and moisturizing effects even after the skin was washed three times with a cleansing surfactant because the skin surface was modified by bactericidal and moisturizing components covalently bonded to the skin surface.

### Comparative Examples 5 and 6 and Examples 19 to 22: Antimicrobial and antistatic detergent composition

Compositions for antimicrobial and antistatic detergents were prepared through a common method of preparing a detergent in compositions and quantities as listed in the following Table 7.

Compositions containing lauryl trimethyl ammonium chloride commonly used as a bactericide in laundry detergents (Comparative Example 5); esterquat commonly used as an antistatic and softening component in laundry detergents (Comparative Example 6); [2-(acryloyloxy)ethyl] trimethyl ammonium chloride capable of surface modification according to the present invention and having both antimicrobial effects and antistatic effects but no cystine reducing agent (Example 19); sodium ascorbate as a cystine reducing agent, and [2-(acryloyloxy)ethyl] trimethyl ammonium chloridete (Example 20); cysteine hydrochloride as a cystine reducing agent, and [2-(acryloyloxy)ethyl] trimethyl ammonium chloride (Example 21); and both sodium ascorbate and cysteine hydrochloride as cystine reducing agents, and [2-(acryloyloxy)ethyl] trimethyl ammonium chloride (Example 22) were prepared.

**[Table 7]**

| Composition (wt%) | Comparative Example 5 | Comparative Example 6 | Example 19 | Example 20 | Example 21 | Example 22 |
|---|---|---|---|---|---|---|
| Water | 68.0 | 68.0 | 66.0 | 65.0 | 65.0 | 65.0 |
| Alkyl sulfate | 15.0 | 15.0 | 15.0 | 15.0 | 15.0 | 15.0 |
| Alcohol ethoxylate | 15.0 | 15.0 | 15.0 | 15.0 | 15.0 | 15.0 |
| Fragrance | 1.0 | 1.0 | 1.0 | 1.0 | 1.0 | 1.0 |
| Lauryl trimethyl ammonium chloride | 1.0 | - | - | - | - | - |
| Esterquat | - | 1.0 | - | - | - | - |
| Sodium ascorbate | - | - | - | 1.0 | - | 0.5 |
| Cysteine hydrochloride | - | - | - | - | 1.0 | 0.5 |
| [2-(Acryloyloxy)ethyl] trimethyl ammonium chloride | - | - | 3.0 | 3.0 | 3.0 | 3.0 |
| Total | 100.0 | 100.0 | 100.0 | 100.0 | 100.0 | 100.0 |

Each of the laundry detergent compositions according to Comparative Examples 5 and 6 and Examples 19 to 22 was used to wash a standard wool fabric with a size of 5 cm x 5 cm ten times to induce surface modification, and each wool fabric was washed three times with a 15% sodium lauryl ethyl sulfate solution (cleansing surfactant) to remove a component which was not covalently bonded but merely adsorbed onto the fabric. Afterward, the wool fabrics thus treated were compared in terms of bactericidal and antistatic effects, the results of which are shown in the following Table 8 (bactericidal ability against E. coli/antistatic effect).

**[Table 8]**

| Classification | Comparative Example 5 | Comparative Example 6 | Example 19 | Example 20 | Example 21 | Example 22 |
|---|---|---|---|---|---|---|
| Bactericidal ability against E. coli (24 hours, %) | 12.5 | - | 97.0 | 99.5 | 100.0 | 99.5 |
| Decrement in static electricity (%) | - | 23.0 | 87.0 | 93.0 | 95.5 | 93.0 |

As shown in Table 8, it was confirmed that Examples 19 to 22 containing the components according to the present invention exhibited excellent bactericidal and antistatic effects even after the wool fabric was washed three times with a cleansing surfactant because the wool fabric surface was modified by functional components covalently bonded to the wool fabric surface.

## Claims

1. A functional composition for surface modification comprising a functional component having a functional group capable of covalently bonding with a thiol (-SH) group of cysteine in a molecule.

2. The functional composition for surface modification of claim 1, wherein the cysteine is included in a protein of hair, skin, fingernails, toenails, a hide, or a fabric.

3. The functional composition for surface modification of claim 1, wherein the functional group capable of covalently bonding with a thiol (-SH) group is an alkene, a vinyl group, or an alkyne.

4. The functional composition for surface modification of claim 1, wherein the functional component is one or more selected from the group consisting of natural extracts, amino acids, peptides, proteins, polymers, silicones, fatty alcohols, fatty acids, waxes, esters, ethers, amines, quaternary ammonium salts, hydrocarbons, alky glyceryl esters, alcohols, polyhydric alcohols, sugars, surfactants, ethylene, derivatives of polyethylene or polypropylene, powders, dyes, antimicrobial agents, insect repellents, fragrances, vitamins, and derivatives thereof.

5. The functional composition for surface modification of claim 1, wherein the functional component having a functional group capable of covalently bonding with a thiol (-SH) group of cysteine in a molecule is one or more selected from the group consisting of [2-(acryloyloxy)ethyl] trimethyl ammonium chloride, pentaerythritol tri(allyl ether), trimethylolpropane di(allyl ether), phosphoric acid 2-hydroxyethyl methacrylate ester, hexanedioic acid divinyl ester, 3,6,9-trioxaundecanedioic acid divinyl ester, butanediol diacrylate, butanediol dimethacrylate, ethylene glycol methyl ether acrylate, ethylene glycol methyl ether methacrylate, poly(ethylene glycol) methyl ether acrylate, poly(ethylene glycol) methyl ether methacrylate, trimethylolpropane triacrylate, pentaerythritol tetraacrylate, dipentaerythritol pentaacrylate, tris(2-hydroxy ethyl) isocyanurate triacrylate, tetra(ethylene glycol) diacrylate, tetra(ethylene glycol) dimethacrylate, polyethylene glycol acrylate, polyethylene glycol diacrylate, polyethylene glycol triacrylate, polyethylene glycol tetraacrylate, polyethylene glycol pentaacrylate, polyethylene glycol hexaacrylate, polyethylene glycol heptaacrylate, polyethylene glycol octaacrylate, polyethylene glycol methacrylate, polyethylene glycol dimethacrylate, polyethylene glycol trimethacrylate, polyethylene glycol tetramethacrylate, polyethylene glycol pentamethacrylate, polyethylene glycol hexamethacrylate, polyethylene glycol heptamethacrylate, polyethylene glycol octamethacrylate, vinyl silicones, alkyne silicones, and derivatives thereof.

6. A functional composition for surface modification comprising:
a functional component having a functional group capable of covalently bonding with a thiol (-SH) group of cysteine in a molecule; and
a cystine reducing agent.

7. The functional composition for surface modification of claim 6, wherein the cysteine is included in a protein of hair, skin, fingernails, toenails, a hide, or a fabric.

8. The functional composition for surface modification of claim 6, wherein the functional group capable of covalently bonding with a thiol (-SH) group is an alkene, a vinyl group, or an alkyne.

9. The functional composition for surface modification of claim 6, wherein the functional component is one or more selected from the group consisting of natural extracts, amino acids, peptides, proteins, polymers, silicones, fatty alcohols, fatty acids, waxes, esters, ethers, amines, quaternary ammonium, hydrocarbons, alky glyceryl esters, alcohols, polyhydric alcohols, sugars, surfactants, derivatives of ethylene or polyethylene, polypropylene, powders, dyes, antimicrobial agents, insect repellents, fragrances, vitamins, and derivatives thereof.

10. The functional composition for surface modification of claim 6, wherein the functional component having a functional group capable of covalently bonding with a thiol (-SH) group of cysteine in a molecule is one or more selected from the group consisting of [2-(acryloyloxy)ethyl] trimethyl ammonium chloride, pentaerythritol tri(allyl ether), trimethylolpropane di(allyl ether), phosphoric acid 2-hydroxyethyl methacrylate ester, hexanedioic acid divinyl ester, 3,6,9-trioxaundecanedioic acid divinyl ester, butanediol diacrylate, butanediol dimethacrylate, ethylene glycol methyl ether acrylate, ethylene glycol methyl ether methacrylate, poly(ethylene glycol) methyl ether acrylate, poly(ethylene glycol) methyl ether methacrylate, trimethylolpropane triacrylate, pentaerythritol tetraacrylate, dipentaerythritol pentaacrylate, tris(2-hydroxy ethyl) isocyanurate triacrylate, tetra(ethylene glycol) diacrylate, tetra(ethylene glycol) dimethacrylate, polyethylene glycol acrylate, polyethylene glycol diacrylate, polyethylene glycol triacrylate, polyethylene glycol tetraacrylate, polyethylene glycol pentaacrylate, polyethylene glycol hexaacrylate, polyethylene glycol heptaacrylate, polyethylene glycol octaacrylate, polyethylene glycol methacrylate, polyethylene glycol dimethacrylate, polyethylene glycol trimethacrylate, polyethylene glycol tetramethacrylate, polyethylene glycol pentamethacrylate, polyethylene glycol hexamethacrylate, polyethylene glycol heptamethacrylate, polyethylene glycol octamethacrylate, vinyl silicones, alkyne silicones, and derivatives thereof.

11. The functional composition for surface modification of claim 6, wherein the cystine reducing agent is one or more selected from the group consisting of ascorbic acid, erythorbic acid, sodium ascorbate, potassium ascorbate, calcium ascorbate, ammonium ascorbate, monoethanolamine ascorbate, diethanolamine ascorbate, sodium erythorbate, disodium ascorbyl sulfate, disodium erythorbyl sulfate, magnesium ascorbyl phosphate, ascorbyl palmitate, ascorbyl stearate, ascorbyl dipalmitate, ascorbyl tetra 2-hexyl decanoate, ascorbyl myristate, ascorbyl laurate, ascorbyl acetate, ascorbyl propionate, ascorbyl tartrate, ascorbyl citrate, ascorbyl succinate, ascorbyl benzoate, potassium (ascorbyl/tocopheryl) phosphate, ethyl ascorbic acid, allantoin ascorbate, chitosan ascorbate, methylsilanol ascorbate, ascorbyl tetradecyl hexyl, aminopropyl ascorbyl phosphate, ascorbic acid polypeptide, ascorbyl glucoside, ascorbyl methylsilanol pectinate, thioglycolic acid, thioglycolate, thioglycolic acid ester, ammonium thioglycolate, sodium thioglycolate, glyceryl thioglycolate, cysteine, cysteine hydrochloride, N-acetyl-L-cysteine, thioglycerol, thiolacetic acid, thiomalic acid, cysteamine, sulfurous acid, ammonium sulfite, sodium sulfite, ammonium hydrogensulfite, sodium hydrogensulfite, thiosulfuric acid, sodium thiosulfate, cystamine, N,N'-bisacryloyl-cystamine, 3,3'-dithiopropionic acid, glutathione disulfide, cysteine hydrochloride, N-acryloyl-cysteamine, 2-iminothiolane, N-acetylhomocysteine thiolactone, 1,4-dithiothreitol, glutathione, and salts and derivatives thereof.

12. The functional composition for surface modification of claim 6, wherein the functional component having a functional group capable of covalently bonding with a thiol (-SH) group of cysteine in a molecule is included in an amount of 0.0001 to 50 parts by weight with respect to 100 parts by weight of the entire composition.

13. The functional composition for surface modification of claim 6, wherein the cystine reducing agent is included in an amount of 0.0001 to 20 parts by weight with respect to 100 parts by weight of the entire composition.

14. A care product comprising the functional composition for surface modification according to claim 1 or 2.
